# DEMANDE DE BREVET EUROPEEN

(11) **EP 3 489 678 A1**
(43) Date de publication de la demande: **29.05.2019**
(21) Numéro de dépôt: 18209007.6
(22) Date de dépôt: 28.11.2018
(51) Int. Cl.: G01N 33/24

(54) **SYSTEME ET PROCEDE DE MESURE DE L'EROSION D'UN SOL**

(30) Priorité: 28.11.2017 FR 1761322
(71) Demandeur: Plotto, Pierre, 38700 Corenc (FR); Université de Nantes, 44000 Nantes (FR); Centre National de la Recherche Scientifique, 75794 Paris (FR)
(72) Inventeur: PLOTTO, Pierre, 38700 Corenc (FR); BENDAHMANE, Fateh, 44600 Saint-Nazaire (FR); MAROT, Didier, 44600 Saint Nazaire (FR); LEROY, Philippe, 44600 Saint Nazaire (FR)
(74) Mandataire: Talbot, Alexandre

(57) **Abrégé**

Système de mesure de l'érosion d'un sol, comprenant un récipient (2) s'étendant le long d'un axe longitudinal (A) et contenant un échantillon (6) du sol, un dispositif d'injection (3) configuré pour injecter un liquide (7) à travers l'échantillon (6), un dispositif de récupération (4) d'une partie (14a à 14d) du liquide injecté, et un dispositif de mesure d'érosion (5) de l'échantillon du sol, le dispositif d'injection (3) comprenant un conduit principal latéral (11) relié à un orifice d'entrée (12) formé dans le récipient (2), le conduit principal latéral (11) étant incliné par rapport à l'axe longitudinal (A) de manière à injecter le liquide (7) à travers l'échantillon (6) selon une direction (D) inclinée par rapport à l'axe longitudinal (A), et le dispositif de récupération (4) comprenant un conduit de récupération latéral (15) relié à au moins un orifice de sortie (16a, 16b, 16c) formé dans le récipient (2).

## Description

### Domaine technique de l'invention

L'invention concerne la mesure de l'érosion d'un sol.

### État de la technique

Il existe des dispositifs de mesure permettant de mesurer l'érosion d'un sol. Le brevet américain US 5331847 divulgue un dispositif comportant une réserve d'eau stockée dans un réservoir extérieur, l'eau contenue dans ce réservoir étant ensuite envoyée sous pression dans un tube creux, le tube creux comportant un trou d'injection dirigé vers le sol dont l'érosion est à mesurer. Avant de réaliser une mesure, le sol est mis à plat, il est ensuite saturé en eau pour éviter que l'eau injectée ne soit absorbée par le sol. De l'eau sous pression est ensuite éjectée, sous forme de jet, contre le sol par le trou d'injection et provoque, en fonction de la vitesse de l'eau, l'érosion du sol au niveau du point d'impact du jet d'eau. Une mesure d'un profil du sol est effectuée puis le volume du sol enlevé par cette eau sous pression est ensuite déterminé en se basant sur les différences entre un profil initial et le profil modifié par l'érosion. Si un tel dispositif permet effectivement de mesurer l'érosion du sol en surface, il ne permet en aucun cas de mesurer l'érosion d'un sol en profondeur. C'est-à-dire à un niveau inférieur à la surface.

On peut également citer la demande de brevet français FR2923015 qui divulgue un dispositif de mesure de l'érosion du sol à un niveau inférieur à la surface, comportant une tête située à une extrémité d'un premier tube creux, le premier tube creux comportant au moins un trou d'injection de liquide situé au-dessus de la tête. Le dispositif comporte en outre des moyens de récupération du liquide injecté comportant un trou de captage du liquide communiquant avec une chambre, et des moyens de transfert, vers un turbidimètre prévu à la surface, du liquide récupéré dans la chambre, les moyens de transfert comportant un second tube creux d'expulsion d'axe sensiblement parallèle au premier tube creux. Mais un tel dispositif nécessite d'introduire la tête à l'intérieur du sol et doit donc être suffisamment solide selon la dureté du sol à tester. En outre, la zone du sol à tester doit être facilement accessible pour placer le dispositif, ce qui n'est pas toujours le cas. Par ailleurs, ce dispositif n'est pas adapté pour mesurer l'érosion d'un sol lorsque le point de sortie de l'eau est situé à distance du point d'entrée de l'eau, ce qui est le cas par exemple pour les digues de retenue. En effet, dans le cas des digues, le point d'entrée de l'eau est situé sur la surface du sol en contact avec l'eau, et en cas d'érosion, la sortie peut être située sur la surface de la digue opposée qui n'est pas en contact de l'eau.

Il existe également des systèmes de mesure utilisés en laboratoire effectuant les mesures à partir d'un échantillon de sol introduit au sein d'un récipient. Mais ces systèmes de mesure analysent d'autres paramètres tels que la perméabilité ou la dureté des sols. Ces systèmes comprennent des moyens d'injection d'un jet d'eau placé au-dessus de l'échantillon pour saturer le sol en eau, mais ces systèmes ne sont pas adaptés pour mesurer l'érosion du sol.

### Objet de l'invention

Un objet de l'invention consiste à pallier ces inconvénients, et plus particulièrement à fournir des moyens de mesure de l'érosion du sol indépendant de l'accessibilité du sol dont on souhaite mesurer l'érosion.

Selon un aspect de l'invention, il est proposé un système de mesure de l'érosion d'un sol, comprenant un récipient s'étendant le long d'un axe longitudinal et contenant un échantillon du sol, un dispositif d'injection configuré pour injecter un liquide à travers l'échantillon, un dispositif de récupération d'une partie du liquide injecté, et un dispositif de mesure d'érosion de l'échantillon du sol.

Le dispositif d'injection comprend un conduit principal latéral relié à un orifice d'entrée formé dans le récipient, le conduit principal latéral étant incliné par rapport à l'axe longitudinal de manière à injecter le liquide à travers l'échantillon selon une direction inclinée par rapport à l'axe longitudinal, et le dispositif de récupération comprend un conduit de récupération latéral relié à au moins un orifice de sortie formé dans le récipient.

Ainsi, on peut mesurer l'érosion d'un sol lorsque le point de sortie de l'eau n'est pas situé à l'aplomb du point d'entrée de l'eau, mais lorsqu'il est décalé par rapport au point d'entrée. Un tel système est particulièrement adapté pour mesurer une érosion lorsque l'eau s'écoule de manière horizontale, oblique ou verticale à travers le sol.

Selon un mode de réalisation, ledit au moins un orifice de sortie est situé au-dessous de l'orifice d'entrée, ainsi on peut facilement récupérer une partie du liquide s'écoulant de manière oblique ou verticale.

Selon un autre mode de réalisation, l'orifice d'entrée et un orifice de sortie sont situés en regard l'un de l'autre sur un même axe perpendiculaire à l'axe longitudinal, ainsi on peut facilement récupérer une partie du liquide s'écoulant de manière horizontale.

Le dispositif de récupération peut comporter plusieurs conduits de sortie ayant, chacun, une première extrémité reliée à un orifice de sortie formé dans le récipient, et une deuxième extrémité reliée au conduit de récupération latéral.

Le dispositif de récupération peut comporter un conduit de réception relié au conduit de récupération latéral et comporte un réservoir rotatif ayant plusieurs compartiments, chaque compartiment étant apte à être placé en regard du conduit de réception à chaque rotation du réservoir.

Le dispositif d'injection peut en outre comporter un conduit axial principal relié à un orifice principal formé dans le récipient, le conduit axial principal étant orienté parallèlement à l'axe longitudinal de manière à injecter le liquide à travers l'échantillon selon une direction parallèle à l'axe longitudinal et le dispositif de récupération comprend un conduit axial secondaire relié à un orifice secondaire formé dans le récipient.

Le conduit axial secondaire peut avoir une première extrémité reliée à l'orifice secondaire et une deuxième extrémité reliée au conduit de réception.

Le récipient peut comporter une paroi latérale dans laquelle l'orifice d'entrée et ledit au moins un orifice de sortie sont formés.

Le système peut également comprendre un réceptacle adapté pour maintenir le récipient en position, le récipient étant en outre configuré pour conserver l'intégrité de l'échantillon.

Selon un autre aspect de l'invention, il est proposé un procédé de mesure de l'érosion d'un sol, dans lequel :
- on fournit un échantillon d'un sol, l'échantillon étant contenu dans un récipient s'étendant le long d'un axe longitudinal,
- on injecte un liquide à travers l'échantillon,
- on récupère une partie du liquide injecté, et
- on mesure une érosion de l'échantillon à partir de la partie du liquide récupérée.

L'injection du liquide à travers l'échantillon est effectuée selon une direction inclinée par rapport à l'axe longitudinal.

On peut récupérer la partie du liquide injecté dans un compartiment d'un réservoir rotatif ayant plusieurs compartiments, à chaque rotation du réservoir.

L'injection du liquide à travers l'échantillon peut en outre être effectuée selon une direction parallèle à l'axe longitudinal.

L'étape de fourniture de l'échantillon peut comporter un forage du sol à l'aide du récipient et une conservation de l'intégrité de l'échantillon au sein du récipient.

### Description sommaire du dessin

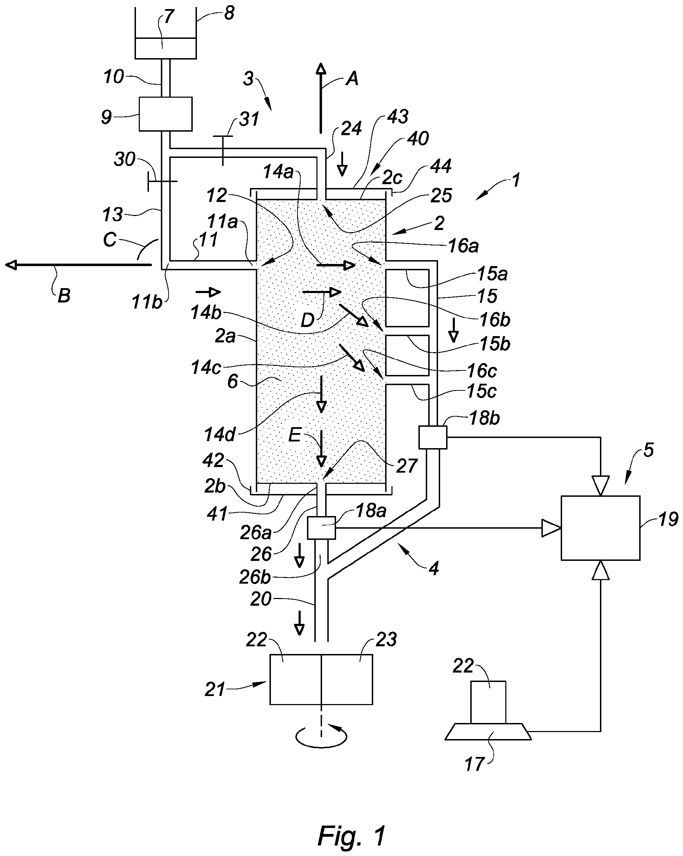

D'autres avantages et caractéristiques ressortiront plus clairement de la description qui va suivre de modes particuliers de réalisation et de mise en oeuvre de l'invention donnés à titre d'exemples non limitatifs et représentés au dessin annexé, dans lequel la figure 1, illustre schématiquement une vue en coupe d'un mode de réalisation d'un système de mesure de l'érosion d'un sol selon l'invention.

### Description détaillée

Sur la figure 1, on a représenté un mode de réalisation d'un système de mesure 1 de l'érosion d'un sol. On entend par érosion, l'action d'un liquide dans un sol engendrant une ablation, un transport ou une accumulation de particules arrachées au sol. La mesure de l'érosion peut comprendre la détermination de la quantité de particules arrachées au sol. Le système de mesure 1 de l'érosion d'un sol comporte un récipient 2, un dispositif d'injection 3, un dispositif de récupération 4 et un dispositif de mesure d'érosion 5.

Le récipient 2 est adapté pour contenir un échantillon 6 du sol dont on souhaite mesurer l'érosion. Le récipient 2 peut être un étui. Le récipient 2 s'étend le long d'un axe longitudinal A. Le récipient 2 comporte une paroi latérale 2a. On entend par paroi latérale 2a, une paroi du récipient 2 s'étendant le long de l'axe longitudinal A. De manière générale, le récipient 2 a une forme d'un cylindre creux. On entend par cylindre, un solide limité par une surface cylindrique engendrée par un ensemble de droites parallèles, notées génératrices, s'appuyant sur une courbe plane fermée, notée directrice, et deux plans coupant les génératrices. Le récipient 2 est configuré pour conserver l'intégrité de l'échantillon 6. C'est-à-dire que l'échantillon 6 n'est pas modifié entre une étape où il est prélevé dans le sol à l'aide du récipient 2, et une étape où il est mis en position au sein du système de mesure 1. De manière générale, le récipient 2 est maintenu en position verticale de manière que son axe longitudinal A soit vertical. Ainsi, l'échantillon 6 est placé dans la même position que la position naturelle du sol à tester. Par exemple, le système de mesure 1 peut comporter un réceptacle 40 destiné à maintenir le récipient 2 en position, c'est-à-dire que le récipient 2 reste immobile par rapport au réceptacle 40. Le réceptacle 40 comporte un socle 41 sur lequel le récipient 2 est posé. Le socle 41 permet de supporter le récipient 2. Le socle 41 peut comporter un bord inférieur 42 en contact avec la paroi 2a du récipient 2. Le bord inférieur 42 du socle 41 entoure une partie inférieure du récipient 2 pour caler le récipient 2 avec le socle 41. Le réceptacle 40 peut en outre comporter une coiffe 43 située à l'opposé du socle 41 et destinée à coopérer avec le socle 41 pour maintenir le récipient 2 en position. Le récipient 2 est alors pris en sandwich entre le socle 41 et la coiffe 43. La coiffe 43 peut comporter un bord supérieur 44 en contact avec la paroi 2a du récipient 2. Le bord supérieur 44 de la coiffe 43 entoure une partie supérieure du récipient 2 pour maintenir efficacement le récipient 2 en position. En outre, le réceptacle 40 peut être fixé à un support, par exemple un mur, pour maintenir l'échantillon en position lors de la mesure de l'érosion. Avantageusement, le récipient 2 comporte un fond 2b et un couvercle 2c pour conserver l'intégrité de l'échantillon 6 lors de son transfert depuis le sol jusqu'au système de mesure. En d'autres termes, le fond 2b et le couvercle 2c permettent de maintenir l'échantillon 6 au sein du récipient entre l'étape de forage du sol et l'étape de mise en position du récipient 2 dans le système de mesure 1.

Le dispositif d'injection 3 est configuré pour injecter un liquide 7 à travers l'échantillon 6. Le liquide 7 peut être l'eau naturelle du site où l'on a prélevé l'échantillon 6, ou une eau de test. Avantageusement, on a préalablement analysé la concentration en particules du liquide 7 afin d'obtenir une mesure de référence de la concentration en particules du liquide 7 utilisé. Le dispositif d'injection 3 comporte une réserve 8, telle une cuve, pour contenir le liquide 7. La réserve 8 est reliée à un contrôleur de débit 9 par un premier conduit 10. Le dispositif d'injection 3 comporte en outre un conduit principal latéral 11 relié à un orifice d'entrée 12 formé dans le récipient 2. L'orifice d'entrée 12 peut être réalisé en forant la paroi latérale 2a du récipient 2 à l'aide d'un foret. L'orifice d'entrée 12 est formé dans la paroi latérale 2a du récipient 2. En particulier, le conduit principal latéral 11 est également relié au contrôleur de débit 9, et donc à la réserve 8, pour permettre une injection du liquide 7 à travers l'échantillon 6. Par exemple, le dispositif d'injection 3 peut comporter un deuxième conduit vertical 13 reliant le conduit principal latéral 11 avec le contrôleur de débit 9. Le conduit principal latéral 11 comporte une première extrémité 11a reliée à l'orifice d'entrée 12, et une deuxième extrémité 11b reliée, soit au contrôleur de débit 9, soit au deuxième conduit 13 comme illustré à la figure 1. De manière générale, la deuxième extrémité 11b du conduit principal latéral 11 est reliée à la réserve 8 pour permettre un transfert du liquide 7 vers l'échantillon 6. Les conduits 10, 11, 13 sont par exemple des cylindres creux. Le conduit principal latéral 11 s'étend le long d'un axe transverse B. Plus particulièrement, le conduit principal latéral 11 est incliné par rapport à l'axe longitudinal A. En d'autres termes, l'axe transverse B est incliné par rapport à l'axe longitudinal A. C'est-à-dire que l'axe transverse B forme un angle C avec l'axe longitudinal A de sorte que l'angle C est strictement inférieur à 180° et strictement supérieur à 0°. Par exemple, l'angle C est compris entre 10° et 170°, de préférence entre 80° et 100°. Avantageusement, l'angle C est égal à 90°. L'inclinaison du conduit principal latéral 11 permet d'injecter le liquide 7 à travers l'échantillon 6 selon une direction D inclinée par rapport à l'axe longitudinal A. Ainsi, on peut mesure une érosion lorsque le liquide s'écoule de manière inclinée par rapport à la verticale.

Le dispositif de récupération 4 est configuré pour récupérer une partie 14a à 14d du liquide 7 injecté. En effet, une première partie du liquide 7 injecté peut être absorbée par l'échantillon 6, tandis qu'une deuxième partie 14a à 14d du liquide 7 circule dans l'échantillon 6. Le dispositif de récupération 4 comprend un conduit de récupération latéral 15 relié à au moins un orifice de sortie 16a, 16b, 16c formé dans le récipient 2. Par exemple, plusieurs orifices de sortie 16a, 16b, 16c sont formés dans la paroi latérale 2a du récipient 2. Préférentiellement, les orifices de sortie 16a, 16b, 16c sont formés sur un côté de la paroi latérale 2a opposé à celui où est formé l'orifice d'entrée 12. Chaque orifice de sortie 16a, 16b, 16c peut être réalisé en forant la paroi latérale du récipient 2 à l'aide d'un foret. Le dispositif de récupération 4 peut en outre comporter plusieurs conduits de sortie 15a, 15b, 15c ayant, chacun, une première extrémité reliée à un orifice de sortie 16a, 16b, 16c formé dans le récipient 2, et une deuxième extrémité reliée au conduit de récupération latéral 15. De préférence, le conduit de récupération latéral 15 s'étend parallèlement à l'axe longitudinal A, et chaque conduit de sortie 15a, 15b, 15c s'étendent perpendiculairement à l'axe longitudinal A. Par exemple, l'orifice d'entrée 12 et un orifice de sortie 16a, noté orifice de sortie principal, sont situés en regard l'un de l'autre. C'est-à-dire que l'orifice d'entrée 12 et l'orifice de sortie principal 16a sont situés sur le même axe transverse B avec l'angle C égal à 90°. En variante, des orifices de sortie additionnels 16b, 16c peuvent être situés entre le fond 2b du récipient 2 et un point d'intersection de la paroi 2a avec l'axe transverse B. C'est-à-dire que certains orifices de sortie 16b, 16c sont situés au-dessous de l'orifice d'entrée 12. Les conduits de sortie 15a à 15c permettent de récupérer respectivement les parties 14a, 14b, 14c du liquide injecté. Un conduit de sortie principal 15a relié à la sortie principale 16a permet de recueillir un écoulement horizontal du liquide 7. Les autres conduits de sortie 15b, 15c permettent de recueillir un écoulement oblique du liquide 7. Ainsi, on peut prendre en compte l'effet de la gravité sur l'écoulement du liquide 7 à travers l'échantillon 6, le liquide 7 ayant tendance à s'écouler vers le fond 2b du récipient 2.

Le dispositif de mesure d'érosion 5 permet de mesurer l'érosion de l'échantillon 6, et d'en déduire une mesure de l'érosion du sol où est extrait l'échantillon 6. La partie 14 du liquide récupérée contient des particules arrachées à l'échantillon 6. Ces particules sont dites fines lorsque leur diamètre est strictement inférieur à 500 micromètres, et sont dites épaisses lorsque leur diamètre est supérieur ou égal à 500 micromètres. Par exemple, on recueil les particules épaisses que l'on pèse, et l'on mesure la turbidité de la partie 14a à 14d du liquide récupérée pour obtenir la concentration du liquide en particules fines. La turbidité d'un liquide correspond au caractère trouble du liquide. La turbidité correspond à la concentration en particules fines du liquide. La mesure de l'érosion est déterminée à partir de la quantité de particules épaisses et fines contenue dans la partie 14a à 14d du liquide récupérée. Le dispositif de mesure 5 comporte une balance 17, des moyens de mesure 18a, 18b de la turbidité d'un liquide, et un ordinateur 19 relié à la balance 17, aux moyens de mesure de turbidité 18a, 18b, au contrôleur de débit 9. L'ordinateur 19 est configuré pour recevoir les mesures transmises par la balance 17, les moyens de mesure 18a, 18b de turbidité, et le contrôleur de débit 9, pour mesurer l'érosion à partir des mesures reçues. De préférence, la mesure de l'érosion effectuée par l'ordinateur 19 peut tenir compte des mesures transmises par le contrôleur de débit 9 pour obtenir une valeur de l'érosion plus précise. La balance 17 permet de peser les particules épaisses. Les moyens de mesure de turbidité 18a, 18b comprennent, par exemple, des capteurs optiques, pour mesurer la concentration de la partie 14a à 14d du liquide en particules fines.

Avantageusement, le dispositif de récupération 4 comporte un conduit de réception 20 et un réservoir rotatif 21. Le conduit de réception 20 est relié au conduit de récupération latéral 15. Le réservoir rotatif 21 comporte plusieurs compartiments 22, 23. Le réservoir rotatif 21 est disposé par rapport au conduit de réception 20, de sorte que chaque compartiment 22, 23 est apte à être placé en regard du conduit de réception 20 à chaque rotation du réservoir 21. Ainsi, on peut mesurer l'érosion dans le temps. En effet, l'érosion d'un sol est modifiée en fonction de la quantité de liquide 7 injecté. En général, au début de l'injection du liquide, l'érosion est importante, puis elle diminue au fur et à mesure que l'on injecte le liquide 7. De préférence, les compartiments 22, 23 sont montés amovibles sur le réservoir 21. Ainsi on peut les extraire du réservoir 21 et les placer sur la balance 17 pour peser les particules qu'ils contiennent.

En outre, le dispositif d'injection 3 peut comporter un conduit axial principal 24 relié à un orifice principal 25 formé dans le récipient 2. L'orifice principal 25 est formé dans le couvercle 2c du récipient 2. Le conduit axial principal 24 est orienté parallèlement à l'axe longitudinal A de manière à injecter le liquide 7 à travers l'échantillon 6 selon une direction E parallèle à l'axe longitudinal A. Le dispositif de récupération 4 comprend en outre un conduit axial secondaire 26 relié à un orifice secondaire 27 formé dans le récipient 2. L'orifice secondaire 27 est formé dans le fond 2b du récipient 2. Le conduit axial secondaire 26 permet de recueillir la partie 14d du liquide qui s'écoule de manière verticale. Le conduit axial secondaire 26 a une première extrémité 26a reliée à l'orifice secondaire 27 et une deuxième extrémité 26b reliée au conduit de réception 20. Le dispositif d'injection 3 comporte en outre des vannes 30, 31 pour ouvrir ou fermer respectivement le conduit principal latéral 11 et le conduit axial principal 24. Les conduits axiaux principal 24 et secondaire 26 sont, par exemple, des cylindres creux.

Un procédé de mesure de l'érosion d'un sol peut être mis en oeuvre par le système de mesure qui vient d'être décrit. Le procédé de mesure comporte les étapes principales suivantes :
- on fournit un échantillon 6 d'un sol, l'échantillon 6 étant contenu dans un récipient 2 s'étendant le long d'un axe longitudinal A,
- on injecte un liquide 7 à travers l'échantillon 6,
- on récupère une partie 14a à 14d du liquide 7 injecté, et
- on mesure une érosion de l'échantillon 6 à partir de la partie 14a à 14d du liquide 7 récupérée.

L'injection du liquide 7 à travers l'échantillon 6 est effectuée selon une direction D inclinée par rapport à l'axe longitudinal A. On récupère la partie 14a à 14d du liquide 7 injecté dans un compartiment 22, 23 d'un réservoir rotatif 21 ayant plusieurs compartiments 22, 23, à chaque rotation du réservoir 21. L'injection du liquide 7 à travers l'échantillon 7 peut en outre être effectuée selon une direction parallèle à l'axe longitudinal A.

La fourniture de l'échantillon 6 peut comporter un forage du sol à l'aide du récipient 2 et une conservation de l'intégrité de l'échantillon 6 au sein du récipient 2. En particulier on conserve l'intégrité de l'échantillon entre le forage et l'injection du liquide 7 à travers l'échantillon 6.

L'invention qui vient d'être décrite permet d'améliorer les mesures de l'érosion d'un sol. Elle permet de prendre en compte des directions variées que peuvent prendre un liquide circulant à travers le sol.

## Revendications

1. Système de mesure de l'érosion d'un sol, comprenant un récipient (2) s'étendant le long d'un axe longitudinal (A) et contenant un échantillon (6) du sol, un dispositif d'injection (3) configuré pour injecter un liquide (7) à travers l'échantillon (6), un dispositif de récupération (4) d'une partie (14a à 14d) du liquide injecté, et un dispositif de mesure d'érosion (5) de l'échantillon du sol, **caractérisé en ce que** le dispositif d'injection (3) comprend un conduit principal latéral (11) relié à un orifice d'entrée (12) formé dans le récipient (2), le conduit principal latéral (11) étant incliné par rapport à l'axe longitudinal (A) de manière à injecter le liquide (7) à travers l'échantillon (6) selon une direction (D) inclinée par rapport à l'axe longitudinal (A), et **en ce que** le dispositif de récupération (4) comprend un conduit de récupération latéral (15) relié à au moins un orifice de sortie (16a, 16b, 16c) formé dans le récipient (2).

2. Système selon la revendication 1, dans lequel ledit au moins un orifice de sortie (16a, 16b, 16c) est situé au-dessous de l'orifice d'entrée (12).

3. Système selon la revendication 1, dans lequel l'orifice d'entrée (12) et un orifice de sortie (16a) sont situés en regard l'un de l'autre sur un même axe (B) perpendiculaire à l'axe longitudinal (A).

4. Système selon l'une des revendications 1 à 3, dans lequel le dispositif de récupération (4) comporte plusieurs conduits de sortie (15a, 15b, 15c) ayant, chacun, une première extrémité reliée à un orifice de sortie (16a, 16b, 16c) formé dans le récipient (2), et une deuxième extrémité reliée au conduit de récupération latéral (15).

5. Système selon l'une des revendications 1 à 4, dans lequel le dispositif de récupération (4) comporte un conduit de réception (20) relié au conduit de récupération latéral (15) et comporte un réservoir rotatif (21) ayant plusieurs compartiments (22, 23), chaque compartiment (22, 23) étant apte à être placé en regard du conduit de réception (20) à chaque rotation du réservoir (21).

6. Système selon la revendication 5, dans lequel le dispositif d'injection (3) comporte un conduit axial principal (24) relié à un orifice principal (25) formé dans le récipient (2), le conduit axial principal (24) étant orienté parallèlement à l'axe longitudinal (A) de manière à injecter le liquide (7) à travers l'échantillon (6) selon une direction (E) parallèle à l'axe longitudinal (A) et le dispositif de récupération (4) comprend un conduit axial secondaire (26) relié à un orifice secondaire (27) formé dans le récipient (2).

7. Système selon les revendications 6, dans lequel le conduit axial secondaire (26) a une première extrémité (26a) reliée à l'orifice secondaire (27) et une deuxième extrémité (26b) reliée au conduit de réception (20).

8. Système selon l'une des revendications 1 à 7, dans lequel le récipient (2) comporte une paroi latérale (2a) dans laquelle l'orifice d'entrée (12) et ledit au moins un orifice de sortie (16a, 16b, 16c) sont formés.

9. Système selon l'une des revendications 1 à 8, comprenant un réceptacle (40) adapté pour maintenir le récipient (2) en position, le récipient (2) étant configuré pour conserver l'intégrité de l'échantillon (6), c'est-à-dire que l'échantillon (6) n'est pas modifié entre une étape où il est prélevé dans le sol à l'aide du récipient (2), et une étape où il est mis en position.

10. Procédé de mesure de l'érosion d'un sol, dans lequel :
- on fournit un échantillon (6) d'un sol, l'échantillon (6) étant contenu dans un récipient (2) s'étendant le long d'un axe longitudinal (A),
- on injecte un liquide (7) à travers l'échantillon (6),
- on récupère une partie (14a à 14d) du liquide injecté, et
- on mesure une érosion de l'échantillon (6) à partir de la partie (14a à 14d) du liquide récupérée,
**caractérisé en ce que** l'injection du liquide (7) à travers l'échantillon (6) est effectuée selon une direction (D) inclinée par rapport à l'axe longitudinal (A).

11. Procédé selon la revendication 10, dans lequel on récupère la partie (14) du liquide injecté dans un compartiment (22, 23) d'un réservoir rotatif (21) ayant plusieurs compartiments (22, 23), à chaque rotation du réservoir (21).

12. Procédé selon la revendication 11, dans lequel, l'injection du liquide (7) à travers l'échantillon (6) est en outre effectuée selon une direction (E) parallèle à l'axe longitudinal (A).

13. Procédé selon l'une des revendications 10 à 12, dans lequel l'étape de fourniture de l'échantillon (6) comporte un forage du sol à l'aide du récipient (2) et une conservation de l'intégrité de l'échantillon (6) au sein du récipient (2).
